# EUROPEAN PATENT APPLICATION

(11) **EP 2 275 444 A1**
(43) Date of publication of application: **19.01.2011**
(21) Application number: 10175623.7
(22) Date of filing: 19.11.2004
(51) Int. Cl.: C07K 16/28, A61P 35/02

(54) **Chimeric anti CD44 antibodies and their use for treating acute myeloid leukemia**

(30) Priority: 19.11.2003 EP 03292873
(62) Divisional of application: 04803792.3
(71) Applicant: Institut National de la Santé et de la Recherche Médicale (INSERM), 75654 Paris Cedex 13 (FR); University Health Network, Toronto, Ontario M5G 2M9 (CA)
(72) Inventor: Smadja, Florence, 92260 Fontenay-aux-Roses (FR); Dick, John E., Toronto Ontario M4E 2Z8 (CA); Kadouche, Jean, 75013 Paris (FR); Boumsell, Laurence, 75015 Paris (FR)
(74) Representative: Peaucelle, Chantal

(57) **Abstract**

The present invention provides two chimeric anti-CD44 antibodies comprising specific animo acid sequence, a (Fab')2, Fab, Fab', scFv, Fv or CDRs fragment thereof, in the preparation of a medicament for eradicating pathological stem cells in cancer therapy, and more specifically in acute myeloid leukaemia therapy.

## Description

The present invention relates to chimeric antibody therapies against cancers, and more specifically against leukaemias.

Different types of leukaemia may be identified: lymphoblastic leukaemias, which particularly comprise acute lymphoblastic leukaemias (ALL) or lymphomas and myeloblastic leukaemias which particularly comprise acute myeloblastic leukaemias (AML). AML represents approximately half of the cases of leukaemia, i.e. approximately 1000 new cases a year in France and 6000 in the USA, with an incidence which increases exponentially over 40 years. AML corresponds to an inhibition of the differentiation of myeloid cells at an immature stage and is conveyed by invasion of the bone marrow and circulating blood by blastic cells, the cytological characteristics of which define the different AML sub-types classified M1 to M7 (French-American-British (FAB) classification), the most frequent being types M1 to M5.

In acute myeloid leukaemia (AML), the leukaemic clone is organized as a hierarchy originating from rare leukaemic stem cells (LSC) with extensive self renewal, which generate leukaemic blasts arrested at various stages of myeloid differentiation, defining the distinct AML subtypes.

In 1978, Leo Sachs published in Nature (1978, Aug 10; 274 (5671) :535-9) that mice leukaemic cells could be induced to differentiate in the presence of physiological growth and differentiation factors. This result was confirmed in human leukaemic cells and successfully transposed in vivo with two differentiation inducers of myelopoiesis, retinoic acid and G-CSF. Unfortunately, despite extensive research, complete remission is obtained in only two AML subtypes (AML3 and AML2 with t(8;21) translocation). The inventors had previously shown (Nat Med. 1999 Jun; 5(6):669-76) that ligation of CD44 reverses the different levels of myeloid differentiation blockage (AML1 to AML5). The differentiation of AML blasts was evidenced by:
- the ability to produce the oxydoreduction function such as oxidative burst,
- the increase expression of lineage antigens, and,
- cytological modifications, all specific of differentiated myeloid cells.

In addition, CD44 ligation with specific monoclonal antibodies (mAbs) can also induce terminal differentiation of THP-1, NB4 and HL60 cell lines, that are interesting models of AML5 (monoblastic subtype), AML3 (promyelocytic subtype)and AML2 (myeloblastic subtype) respectively. A massive apoptotic cell death could then be induced in NB4 cells but only a very moderate one in THP-1 and HL60 cells.

The leukaemic stem cells (LSC) are distinguished from all other AML cells by self renewal ability, i.e. the ability to generate daughter cells similar to the mother one. The extensive self-renewal ability is an intrinsic property of LSC, and has been shown essential for the development of leukaemia.

Experimentally, the human LSC are identified by transplantation into NOD/SCID immunodeficient mice in which they generate a disease faithfully recapitulating the AML type of the donor. Since they possess the ability to initiate the leukaemic clone on transplantation, they have been termed the SL-IC for SCID-Leukaemia Initiating Cells. These SLC are distinct from the other leukaemic cells because they are exclusively present within a CD34+cd38- cell fraction representing from 0.1% to 1% of the AML cells population, and this is true in all AML subtypes.

In summary, in order for new therapies to cure AML, the LSC must be effectively targeted and eradicated.

The conventional treatment of AML is chemotherapy but, although it succeeds to induce an initial complete remission in 60-85% of patients, it is still unable to cure most AML patients (5-years survival rate: 37%) and only little progress has been made in the long-term survival of AML patients, especially in adults over 55-60 years (5-years survival rate: 15%). This situation has prompted efforts to develop new targeted therapeutic approaches, using pro-apoptotic agents (arsenic trioxide), anti-sense strategies (anti BCL2) and anti-inducers of transcription (DNA methylases, histone acetylating agents). However, most therapeutic strategies currently employed, target cycling cells, and SL-IC are quiescent, indicating that new approaches must be found.

Previous works of the inventors arose a new therapeutic treatment specifically targeting leukaemic cells to induce differentiation of these cells, involving an anti-CD44 antibody (Charrad et al. Nature Medicine 1999). Such a therapy, called « differentiation therapy » has been used to treat patients with AML3 subtype, using retinoic acid as differentiation-inducing molecule, and in combination with the conventional treatment, which is chemotherapy.

Further experimentations lead the inventors to select the specific anti-CD44 antibody, H90 (also named P245), capable of not only differentiating leukaemic cells but also eradicating leukaemic stem cells. In fact, this anti-CD44 antibody completely cured human leukaemic NOD/SCID mice (Jin L. et al. 2003). Since the chemotherapy is a painful treatment, this antibody is likely to replace chemotherapy by being used alone in a monotherapy.

However, this antibody being a murine anti-CD44 antibody, its use in humans will be limited. In fact, murine antibodies injected can result in a response by the patient against this foreign protein, known as HAMA (Human Anti-Mouse Antibodies). This development of HAMA can be variable from one antibody to another, but, once HAMA have appeared, the administration of murine antibodies loses all effectiveness.

The inventors have now developed chimeric anti-CD44 antibodies having improved activities, in particular, an improved differentiation activity.

Accordingly, the present invention then provides two chimeric anti-CD44 antibodies useful in monotherapy against cancers, and more specifically against leukaemias.

The two chimeric anti-CD44 antibodies according to the invention comprise amino acid sequences coded by nucleotide sequences SEQ ID n°1, 3 and 5, or SEQ ID n° n°1, 3 and 7 respectively, the corresponding amino acid sequences being SEQ ID n°2, 4 and 6, and SEQ ID n°2, 4 and 8, respectively. The invention also covers F(ab')2, Fab, Fab', scFv, Fv or CDRs (Complementary Determining Regions) fragments thereof and the nucleotide sequence SEQ ID N°1, SEQ ID N°3, SEQ ID N°5, the amino acid sequence SEQ ID N°2, SEQ ID N°4, SEQ ID N°6 and the transfer construct comprising at least one of said nucleotide sequences.

The first chimeric anti-CD44 antibody or fragments thereof refers to an anti-CD44 antibody chimerized with a gamma 1.

This gamma 1 construct can provide, in addition to the initial activity, a strong ADCC (antibody dependent cell-mediated cytotoxicity) and CDC (Complement-Dependent Cytotoxicity) component.

On the contrary, the second chimeric anti-CD44 antibody or fragments thereof corresponds to a gamma 4 construct with little or no ADCC and CDC. Moreover, this latter construct also has the advantage of having a weak carbohydrate component, which allows us to avoid certain reactions, in particular heteroimmunization reactions, or non-specific binding. Furthermore, due to this particular neutrality regarding immunotoxicity and in addition to its own differentiating therapeutic activity, the gamma 4 construct could be used with benefit as a targeting molecule on which could be linked any complementary therapeutic medicine or particle (radioisotopes, cytotoxic compounds, ...)

Additionally, the gamma 4 chimeric antibody half-life is of the order of 20 to 25 days, which is entirely satisfactory.

Advantageously, the expression of such antibodies using recombinant baculoviruses is completely controlled regarding the sugar composition.

Preferably, the whole chimeric antibodies are used. In fact, a therapeutic antibody is usually more effective when used as a whole antibody rather than F(ab')2, Fab or Fab' fragmented antibody or in the form of small constructs (scFv, etc).

In another preferred embodiment, the chimeric antibodies are monoclonal antibodies.

The invention further relates to the use of the chimeric anti-CD44 antibodies according to the invention in the preparation of a medicament for eradicating pathological stem cells in cancer therapy.

The invention further relates to the use of the chimeric anti-CD44 antibodies according to the invention in the preparation of a medicament for specifically eradicating pathological stem cells and preserving normal stem cells *ex vivo* in cancer therapy.

Another aspect of the invention concerns a method for eradicating pathological stem cells from a patient, comprising administering to said patient, a chimeric anti-CD44 antibody according to the invention, under conditions allowing an antigen-antibody reaction, such that only pathological stem cells are eradicated.

Moreover the present invention further concerns a method for specifically eradicating pathological stem cells and preserving normal stem cells *ex vivo* from a leukaemic or cancerous patient's tissue sample, comprising contacting said tissue sample with at least one chimeric anti-CD44 antibody according to the invention under conditions allowing an antigen-antibody reaction. Such a method is adapted for purifying bone marrow cell populations.

Said medicament/method avoids the generation of pathological cells issued from pathological stem cells, in particular, leukaemic cells and cancer cells.

As mentioned above, a differentiation therapy needs the use of a chemotherapy in order to eradicate leukaemic stem cells. The use of the chimeric anti-CD44 antibodies, according to the invention, not only induce the differentiation of leukaemic cells with an improved efficiency but also induce the eradication of leukaemic stem cells, without the requirement of any chemotherapy.

The medicament according to the invention may be administered at doses from approximately 10 mg to 1000 mg by cure, preferably in the order of 100 to 400 mg. The number of cures may be increased or reduced and/or repeated (over time) to improve the efficacy of the medicament.

Since the chimeric gamma 4 antibody is not toxic, its dosage may be easily increased and adapted to the patient.

The production of the medicament may be in any suitable pharmaceutical formulation, and particularly in the form of tablets, granules, capsules, powder forms, suspension, oral solutions, solutions for injection. Administration may be preferably performed by slow infusion.

The medicament, used according to the invention, may also further comprise any suitable compound or excipient adapted to the desired formulation, particularly any pharmaceutically inert vehicle.

Advantageously, a suitable formulation is a saline solution for injection, preferably intravenous injection.

Pathological cells that can be treated by the medicament according to the invention are leukaemic cells, pathological stem cells, and more particularly leukaemic stem cells and breast or colon cancer stem cells.

Indeed, there is increasing evidence that in other cancers, like in AML, the tumor clone is also maintained by the extensive proliferation and self-renewal of rare tumor stem cells. Since CD44 is also present in most cancer cells, CD44 ligation may be also efficient to eradicate such tumor stem cells, and thereby, it may have a therapeutic effect also in several cancers other than AML.

The present invention is illustrated by the following examples, given for purely illustrative purposes, with reference to the following figures:
- Figure 1 refers to the Southern Blot of the DNA of 4 viral clones: B2954, B2955, B2957, B2958. The first, second and third slides correspond respectively to the BET coloration, the hybridisations with a Cγ1 probe and with a kappa probe.
- Figure 2 refers to the Southern Blot of the DNA of another 4 viral clones: B3667, B3668, B3669, B3670. The first, second and third slides correspond respectively to the BET coloration, the hybridisations with a kappa probe and with a gamma probe.

The present invention also comprises any alternative embodiment that may be produced by those skilled in the art, without undue experimentation, from the disclosure given by the present application (including disclosure, examples, claims and figures) and means according to the prior art.

### Example 1: Anti-CD44 monoclonal antibodies eradicate leukaemic (tumor) stem cells

NOD/SCID mice leukaemia model and transplanted PML-RAR mice are used. The NOD/SCID mice leukaemia model is a unique model, that faithfully recapitulates the pathology of *all subtypes* (except AML3) of human AML, and, most importantly, allows to monitor the fate of the very small subpopulation of human leukaemic stem cells, endowed with extensive proliferation and self-renewal capacity, and responsible for the maintenance of the leukaemic clone.

### Materials and Methods

**AML cells.** Fresh or frozen AML peripheral blood cells were enriched by Ficoll-density gradient centrifugation and washed in Iscove's Modified Dulbecco's medium (IMDM) containing 5% fetal calf serum.

**Transplantation of AML cells into NOD/SCID mice.** 8- to 12-week-old NOD/SCID mice are sub-lethally irradiated with 375 or 400 cGy from a ¹³⁷Cs source immediately before tail vein injection of AML cells. Mice receive human stem cell factor (SCF) and a fusion protein of huIL3/hu GM-CSF (PIXY321) every other day as intraperitoneal injections at a concentration of 10µg and 7µg per mouse, respectively.

**Assay for leukaemic stem cells (LSC).** It has been demonstrated (Bonnet and Dick, Nature Medicine 3:730-737, 1997) that the engraftment of AML into NOD/SCID mice results from proliferation and limited differentiation of a rare population of leukaemic stem cells (LSC), displaying a CD34++ CD38neg immunophenotype, that is present in the leukaemic clone and sustain it. Therefore, the success of the AML engraftment demonstrates the presence of LSC. At indicated time points (4-8 weeks), the percentage of leukaemic infiltration in bone marrow of transplanted NOD/SCID mice is evaluated by aspiration from the knee joint (average 10⁶ per aspirate) at different time points. The leukaemic population is labeled using a panel of mAbs to haematopoietic-specific antigens (CD45) and differentiation antigens (CD33, CD14, CD15, CD11b). The absence of CD19 is considered as indicator that the differentiated cells do not originate from *normal* haematopoietic stem cells comprised in the grafted AML sample.

### Results

**Table 1: P245 inhibits the development of AML stem cells in NOD/SCID mice. Mice were intravenously injected with 15.10⁶ human AML cells (day 0), and treated with P245 from day 20 to day 50 (750µg/injection, 3times per week). The % of human AML cells was measured in the bone marrow, on the basis of human pan-myeloid antigen huCD45 expression (aspiration from the knee joint, average 10⁶ cells per aspirate). Data are means +/-SD from 3 independent experiments, 5 mice /group. This table shows that P245 inhibits the development of AML. * secondary recipients did not receive P245 injection**

| | | % huCD45+ cells | | | |
|---|---|---|---|---|---|
| | | in primary recipients | | in secondary recipients* | |
| Patient | AML subtype | untreate d | P245-treated | From untreated primary recipient | from P245-treated primary recipient |
| 4971 | M5 | 23+/-19 | 0 | 23.4+/-16 | 0.0+/-0.1 |
| 5131 | M5 | 67+/-20 | 7+/-10 | 1.7+/-1.5 | 0.0+/-0.1 |
| 5173 | M4 | 14+/-12 | 2+/-2 | 7.3+/-3 | 0+/-0 |

The four independent experiments performed so far clearly show, in a very reproducible manner, that P245 is highly efficient to eradicate most AML cells in the primary recipients (table 1). This may be partly due to the induction of terminal differentiation, as shown in table 2. However, it is also, and probably mainly, due to the eradication of most leukaemic stem cells, as shown by secondary transplantation assays (table 1). These results show that it is possible to eradicate AML stem cells *in vivo,* and it should be pointed out that no toxicity nor other undesirable side-effect was observed. The effect of P245 on long-term survival was further investigated. In addition, the effect of P245 on normal stem cells was also studied. Most interestingly, in a preliminary experiment, no inhibitory effect of P245 on the engraftment of normal CD34+ cord blood cells was observed, showing that P245 selectively eradicate AML stem cells in vivo.

**Table 2. In vivo differentiation of AML blasts in P245-treated primary recipients: Differentiation is evidenced by increased expression of the granulocytic-specific differentiation antigen CD15, on the AML cells (CD45+). This experiment is one representative of 4 independent experiments.**

| Patient | Treatment | % huCD45+ cells | %CD15+in the CD45+ population |
|---|---|---|---|
| 4971 | No | 43 | 22 |
| | P245 | 6.2 | 56 |

The transplanted PML-RAR mice, has allowed the inventors to investigate the in vivo effect of CD44-targeted molecules on a model of AML3 subtype, the only one which can not be engrafted into NOD/SCID mice. Since mAbs to murine CD44 was not at disposal, the therapeutic efficacy of HA was investigated, and compared to the one of retinoic acid, which induces full terminal differentiation of AML blasts and full remission of the transplanted PML-RAR mice. The results obtained (summarized in table 3), clearly show that, after 4 days of administration, HA is as efficient as retinoic acid to abrogate the splenomegaly characteristic of the disease, and it also succeeds to decrease leukaemic blast infiltration in the bone marrow. This effect is HA-dose dependent. The apparition of differentiated granulocytic precursors strongly suggest that HA induces terminal differentiation of AML blasts, as it does in vitro, and similarly to retinoic acid. Collectively these results show that for the first time CD44 ligation is an efficient means to eradicate AML cells in vivo and provide a new basis for developing CD44 targeted therapy in AML.

**Table 3: HA inhibits growth and induces terminal differentiation of PML-RAR cells (AML3) in vivo HA (6.105kAa) was administered through an osmotic pump, at a rate of 1µl/hour for 4 days, into leukaemic mice, engrafted 12 days before with 10⁵ leukaemic PML-RAR blasts. A strong inhibition of splenomegaly is observed, associated with a decrease of blastic infiltration (enumerated by microscopic observation) in the bone marrow and an increase of differentiating granulocytic precursor cells. Data are means +/-SD from 3 independent experiments, 5 mice /group. RA: retinoic acid**

| Treatment | mean spleen weight (mg) | % blasts in bone marrow | % myelocytes plus metamyelocytes in bone marrow |
|---|---|---|---|
| No | 480+/-45 | 85+/-7 | 12+/-3 |
| HA | 120+/-57 | 28+/15 | 48+/-23 |
| RA | 135+/-68 | 18+/-7 | 67+/-17 |

### Example 2: Chimerization and expression of the murine antibody H90 in the baculovirus-Sf9 cell system - Expression of an IgG1

### Starting material

Two pellets of 5 × 10⁶ cells of the H90 hybridoma (on dry ice) were used. The H90 monoclonal antibody is an IgG1 kappa.

« Baculomab » technique was used as described in WO 95 20672 and in the article by Lieby et al., Blood, 15 june 2001, vol. 97, n°12, p.3820-3828.

### 1 - Isolation of the heavy and light variable regions

- **Isolation of total RNA:** the Qiagen extraction kit (Ref. 74 104) was used according to the protocol described by the supplier.
- **Complementary DNA synthesis**

A reverse transcription was carried out on the total RNA extracted above. The transcriptase used is Omniscript from Qiagen (Ref. 205 111) .
- **Amplification of VH and VL**
   The technology used is derived from that which is described in *"*Chardès, T., Villard, S., Ferrieres, G., Piechaczyk, M., Cérutti, M., Devauchelle, G., and B. Pau. 1999. Efficient and direct sequencing of mouse variable regions from any immunoglobulin gene family. FEBS Lett. 452, 386-394***"***
   To identify and clone the light or heavy variable region, 18 PCRs are carried out in parallel. The fragments of approximately 400 bp are then cloned into the plasmid pGEMTeasy and then sequenced. This sequencing is performed by the company MWGBiotech.

### a - Amplification of the VL region

### Results

1st trial: 2 clones were studied
   - H90 32LS Cl 15
   - H90 32LS Cl 18
   Analysis of the sequences shows that clones 15 and 18 do not encode immunoglobulin variable regions. A further preparation of complementary DNA was therefore carried out (modified protocol) and the PCR products were cloned and analyzed.
2nd trial: 4 clones were obtained
   - H90 3LS Cl 5
   - H90 3LS Cl 7
   - H90 22LS Cl 12
   - H90 22LS Cl 16
   Clones 12 and 16 correspond to a kappa pseudogene (*Mus musculus immunoglobulin aberrantly rearranged kappa chain mRNA*).
   Clone H80 3LS Cl 7 indeed corresponds to a functional VL-kappa variable region (SEQ ID N°1 and 2). This clone will therefore be inserted into the baculovirus transfer vector pBHUCk47.

### b - Amplification of the VH region

The same technique is used to isolate the heavy variable regions. 18 PCRs are performed.

### Results

1st trial: 1 clone was analyzed
   - H90 19HS Cl 9
   Analysis of the sequence of this clone shows that it corresponds to a murine pseudogene (reading frame shift).
2nd trial: a new complementary DNA was synthesized and used for the PCR analysis.
   2 clones were analyzed:
   - H90 17HS Cl 23
   - H90 17HS Cl 28

These two clones indeed correspond to a murine gamma 1 variable region; unfortunately, approximately 1/3 of the N-terminal sequence was missing. Analysis of the sequence on IMGT enabled us, however, to identify the most probable germinal line of origin and therefore to design other PCR primers.

By virtue of the latter approach, the inventors isolated a new clone: H90 3HS clone 15.

Analysis of the sequence shows a correct murine VH region (SEQ ID N° 3 and 4). This is the clone which was introduced into the baculovirus transfer vector.

### 2 - Insertion of the heavy and light variable regions into the transfer vectors

PCR primers were designed in order to amplify these VHs and VLs and to allow their insertion in phase with the signal sequences located upstream and the kappa or gamma 1 constant regions located downstream.

The constructs obtained were again verified by sequencing.

### 3 - Construction of the recombinant virus expressing the H90 antibody in the form of an IgG1

The heavy region of the H90 antibody was isolated from the plasmid construct H90 3HS clone 15, and then inserted into a transfer vector p119Cg1 that allows the expression of a human γ1 heavy chain. The sequence of the gamma 1 constant region is given (SEQ ID N°5 and 6).

### 4 - Obtaining of the recombinant virus

Sf9 cells were cotransfected with (i) purified viral DNA and (ii) the DNA of the two transfer vectors containing the VH and VL regions described above (SEQ ID N°1 and 3).

The viruses generated after incubation for 5 days at 28°C were cloned by means of the lysis plaque technique. Nine isolated clones were amplified, and antibody expression was verified by ELISA. All the clones were found to be positive. The clones B2954, B2955, B2957 and B2958 were chosen for the complementary analyses.

### 5 - Verification of the specificity of the antibody produced

The 4 viral clones expressing the antibody were re-amplified and approximately 5 ml of culture supernatant analyzed in order to verify the specificity of the recombinant antibody for human CD44.

### 6 - Verification of the genome of the recombinant viruses

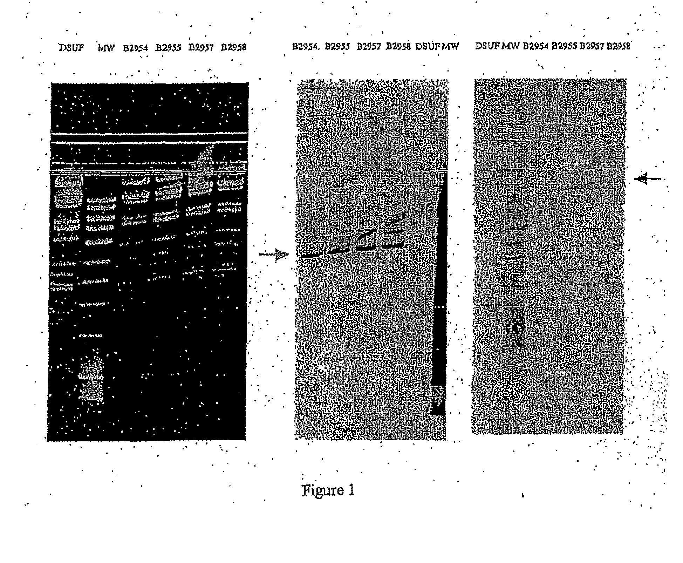

The DNA of the 4 viral clones was extracted, digested with HindIII restriction endonuclease, and then analyzed by Southern blotting. Two probes specific for the heavy and light chains were used successively: a Cγ1 probe and a kappa probe (figure 1).

### Example 3: Chimerization and expression of the murine antibody H90 in the baculovirus-Sf9 cell system - Expression of an IgG4

The VH and VL variable regions of a murine anti-CD44 antibody were isolated from the H90 hybridoma as described in example 2.

### 1 - Construction of the recombinant virus expressing the H90 antibody in the form of an IgG4

The heavy region of the H90 antibody was isolated from the plasmid construct H90 3HS clone 15, and then inserted into a transfer vector p119Cg4 that allows the expression of a human γ4 heavy chain. The sequence of the gamma 4 constant region is given (SEQ ID N°7 and 8) .

### 2 - Obtaining of the recombinant virus

Sf9 cells were cotransfected with the 2 transfer vectors (recombinant baculovirus) containing, respectively, the VH (p119Cg4) and VL (pBHUCk47) variable regions described above, and viral DNA. After incubation for 5 days at 28°C, the viruses generated were cloned by means of the lysis plaque technique. Ten isolated viral clones were amplified. Antibody production was verified by ELISA (capture antibody: anti-Fdγ1, The Binding Site, visualization with a peroxidase-labelled human anti-kappa antibody, Sigma).

**ELISA result:** All the isolated viral clones produce antibody.

| | **IgG4 H90** | | |
|---|---|---|---|
| **PBS** | **16 clones: B3651 to B3666** | | **+ Control** |
| 0.08 | **B3651** | **1.245** | 3.314 |
| 0.071 | B3652 | 0.737 | 3.065 |
| 0.083 | B3653 | 0.723 | 2.484 |
| 0.067 | **B3654** | **0.909** | 1.759 |
| 0.057 | B3655 | 0.874 | 1.127 |
| 0.056 | B3656 | 0.778 | 0.707 |
| 0.058 | B3657 | 0.837 | 0.413 |
| 0.071 | B3658 | 0.809 | 0.257 |
| 0.116 | **B3659** | **0.99** | |
| 0.07 | B3660 | 0.743 | |
| 0.058 | B3661 | 0.869 | |
| 0.055 | B3662 | 0.729 | |
| 0.064 | B3663 | 1.021 | |
| 0.053 | B3664 | 0.784 | |
| 0.077 | **B3665** | **1.45** | |
| 0.078 | B3666 | 0.733 | |

| | | | |
|---|---|---|---|
| In bold: 4 clones selected | | | |

### 3 - Verification of the genome of the recombinant viruses

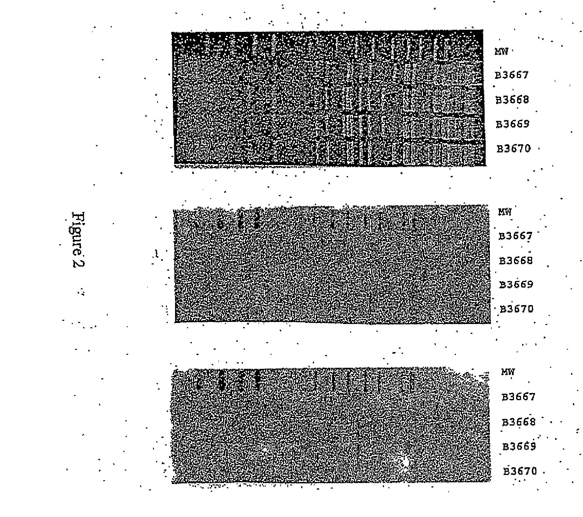

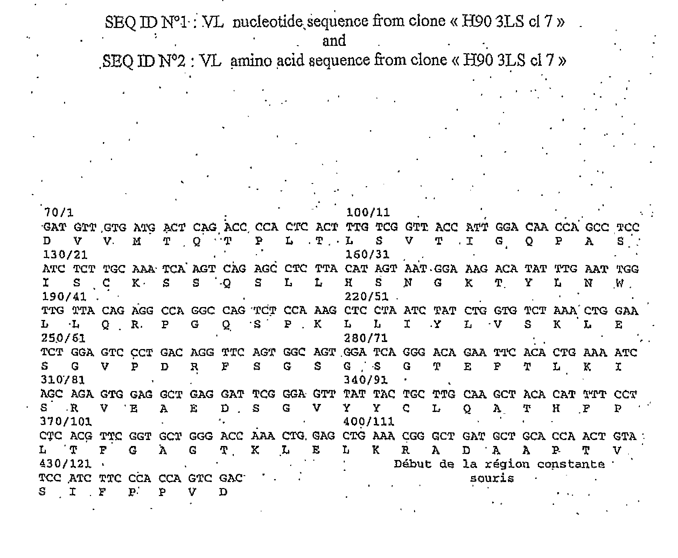

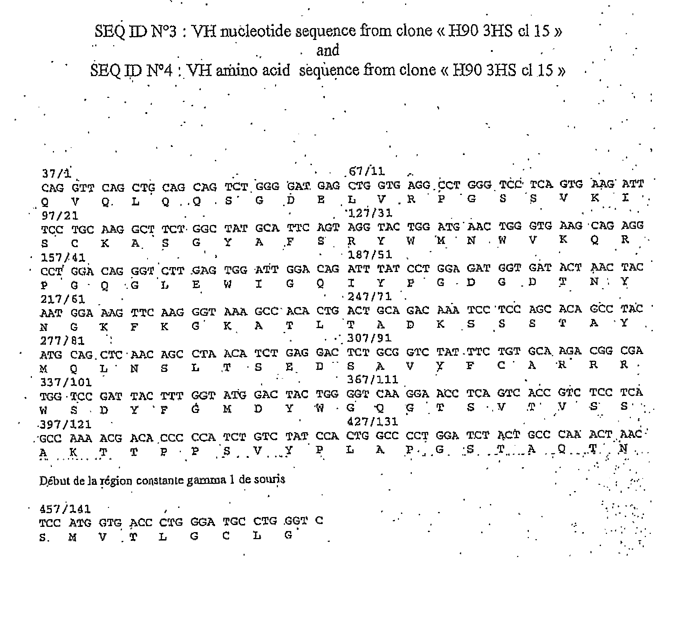

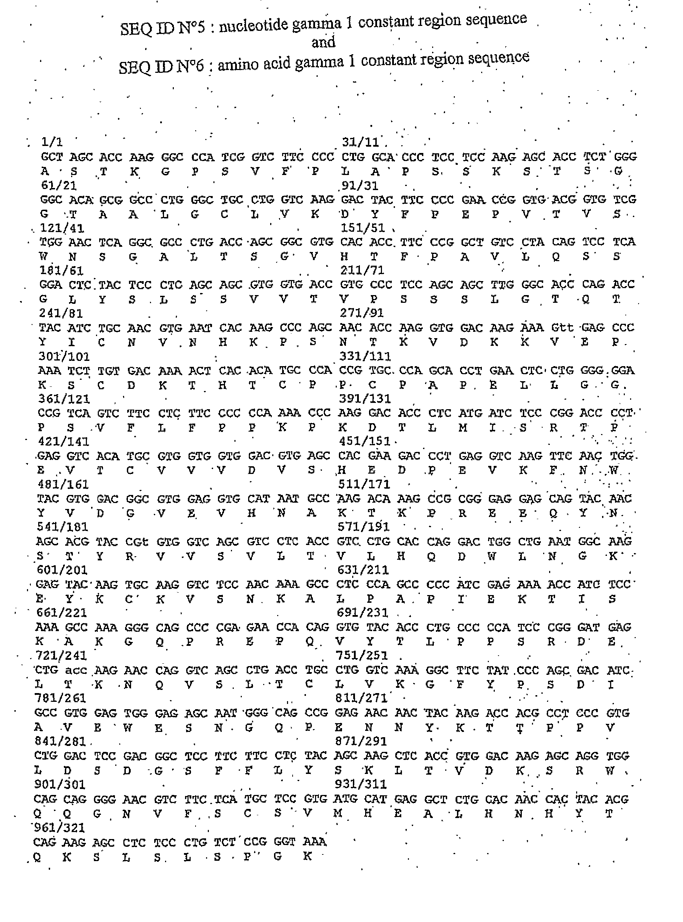

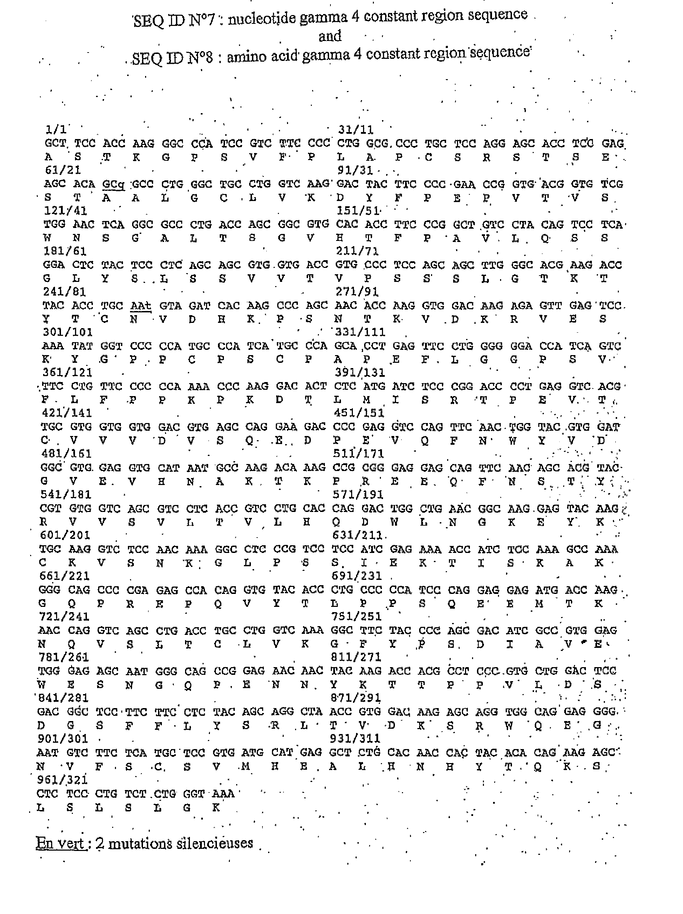

The genomes of 4 viral clones expressing the antibody were verified (clones B3667, B3668, B3669 and B3670). After amplification, the viruses contained in the culture supernatants were sedimented at 35 000 rpm for 30 minutes (TLA 100, Beckmann). The DNA of these viruses were subsequently extracted and purified, and then digested with the HindIII restriction endonuclease. The digestion products were separated on an agarose gel and then hybridized with two probes specific for human heavy and light chains (figure 2)

Two of these clones, B3668 and B3669, exhibit restriction and hybridization profiles corresponding to the expected genomic organization. A single recombinant, B3669, was amplified and then titred for the production and the purification of the recombinant H90γ4 antibody.

The specificity of the antibody with respect to CD44 was verified.

### 4 - Antibody production and purification

Sf9 cells were adapted to culture in serum-free medium (SB5/6 medium, supplied by Dr Gérard Devauchelle (Saint Christol Les Alès, France).

For production, the Sf9 cells are seeded at a density of approximately 500 000 cells/ml in rolling bottles in a final volume of 400 ml. The cells are immediately infected with the B3669 virus at a multiplicity of infection of 2 PFU/cell, and then incubated at 28°C.

After 4 days of infection, the cultures are centrifuged at 3000 rpm. The supernatants are removed and stored at -80°C until use.

The antibodies contained in the culture supernatant are purified according to the conventional technique of purification on protein A. Briefly, the pH of the culture medium is adjusted to 8.5 and this medium is then filtered through a 0.5 µm Millipore cartridge before being loaded onto a protein A column (High Trap ProtA FF, Amersham). After the column has been washed with a 100 mM Tris buffer, pH 8.5, the antibodies are eluted in 100 mM sodium citrate, pH 3.0. The fractions collected are neutralized with 2M Tris, pH 8.8 (1 volume of Tris per 3 volumes of eluate).

The antibody is concentrated on Macrosep 30K (Pall). The preparation is assayed by ELISA and analyzed by migration on a 10% polyacrylamide gel and silver staining.

### Example 4: Differentiation activity of the chimeric anti-CD44 antibodies.

### H90 chimerized with human gamma 1 / gamma 4

The study of the differentiating activity of H 90 chimerized with human gamma 1 was performed on the myeloid leukaemia cell lines THP-1 and NB4. This study was carried out using the differentiation criteria already described in the publications by Florence Smadja-Joffe (ref Blood January 2002, vol 99, number 1 - Blood August 2000, vol 96, number 3). This previous study has shown that a most reliable differentiation parameter is the increase in the level of myeloid-differentiation specific antigens such as CD11b (increased during both granulocytic and macrophagic differentiation), CD14 (monocytic-differentiation specific) and/or CD15 (mostly increased in the course of the granulocytic differentiation but also, to a lower extent, in the course of the monocytic one).

### Results

THP-1 and NB4 cells are established cell lines which are models for AML5 and AML3 subtypes, respectively (Charrad et al Blood 2002). These cell lines have been treated for 3 days with different forms of the differentiation-inducing anti-CD44 antibody H90: the murine one, or the one chimerized with a human gamma 1 chain, or the one chimerized with a human gamma 4 chain (designated as ch-human gamma 1 chain and ch-human gamma 1 chain H90, respectively). Each one of these forms has been used at the final concentration of 50µg/mL. The level of differentiation antigens CD11b, CD15 and CD14 has been evaluated by using FITC(fluorescein isothiocyanate)-conjugated specific monoclonal antibodies and flow-cytometry analysis. It is expressed by the percentage of positive cells, and by the mean fluorescence intensity, all relative to the ones of cells labelled with FITC-conjugated isotype-matched IgG. The distinct forms of H90 were considered to be capable to induce differentiation if they provoked an increased expression of at least 1 or 2 differentiation antigens, of either the percentage of positive cells or the mean fluorescence intensity.

The results shown in table 4, indicate that both human gamma 1 and human gamma 1 chimerized H90 molecules are more efficient that the murine one to inducing differentiation of both THP-1 and NB4 cells. Indeed, in THP-1 cells, they provoke a higher increase of both the percentage of CD15 positive cells and of the CD15 fluorescence intensity, and a higher increase of the CD14 positive cell percentage. Similarly, in NB4 cells they increased the expression of CD15 than the murine H90 form, and they were as efficient as the murine H90 to increase the level of CD11b; human gamma 4 chimerized H90 was, in this regard, more efficient than human gamma 1 chimerized H90.

**Table 4**

| | | | Controls* | H90 | | |
|---|---|---|---|---|---|---|
| | | | | murine | ch-human gamma 1 chain** | ch-human gamma 4 chain** |
| **THP-1 cells (monoblastic AML cells)** | CD15 | **% positive cells** | 87 | 72 | 99 | 83 |
| | | **Mean fluorescence intensity** | 36 | 45 | 94 | 65 |
| | CD14 | **% positive cells** | 0 | 3 | 11 | 14 |
| | | **Mean fluorescence intensity** | 0 | 28 | 22 | 25 |
| | | | | | | |
| **NB4 cells (promyelocytic AML cells)** | CD15 | **% positive cells** | 87 | 72 | 99 | 83 |
| | | **Mean fluorescence intensity** | 36 | 45 | 94 | 65 |
| | CD11b | **% positive cells** | 19 | 33 | 35 | 34 |
| | | **Mean fluorescence intensity** | 32 | 47 | 27 | 48 |

| | | | | | | |
|---|---|---|---|---|---|---|
| Controls*: cells treated with murine IgG1, human gamma1, or human gamma4. similar values were obtained. Values shown in this table are those obtained by treating the cells with murine IgG1. | | | | | | |

Added to this are the CDC cytotoxic activity and especially the ADCC cytotoxic activity induced by the Fc portion of the gamma 1 construct.

The cytotoxic activity of chimeric P245 (H90) were studied *"in vitro"* on the 2 AML lines previously described and compared with other anti-CD44 antibodies, including P245, A3D8 and Hermes 3.

On THP1 and NB4, a strong cytotoxic capacity of the chimerized gamma 1 antibody was observed. At day 4, the cytotoxicity is greater than 80%. This activity manifests itself only with the chimerized antibody; none of the murine antibodies possess this property. This is also observed with cells from patients suffering from AML5.

Monoblastic-type AML5 cells are both target cells, since they express the CD44 receptor, and effector cells since, as monocytes, they possess an Fc receptor for gamma-globulins, especially gamma 1, engendering the ADCC phenomenon, which is not the case of promyelocytic AML3 cells, which are only target cells.

This ADCC activity was also studied *"in vitro"* using NK cells (conventional assay) for cytotoxicity.

For the gamma-4 construct, only differentiating activity, without cytotoxicity, was observed.

### Example 5: Formulation of a galenic vector - emulsion

The LIPOID E-80, Vit E and stearylamine are dissolved directly in the oil phase. Whereas the poloxamer and glycerol are directly dissolved in the aqueous phase. The oil and aqueous phases are prepared separately using a magnetic stirrer, filtered and heated to a temperature of 70°C. The two phases are mixed using a magnetic stirrer. The temperature is brought up to 85°C. The mixture is homogenized with Polytron or Ultraturrax for 3 to 5 min. The temperature is decreased rapidly to 20°C. The emulsion is passed trough a high-pressure homogenizer (microfluidizer) for 5 min. The temperature is brought rapidly to 20°C. The pH is adjusted to the desired value with 0.1M hydrochloric acid. The emulsion is filtered through a 0.45µm filter, stored under nitrogen atmosphere in siliconized glass bottles and sterilized in an autoclave.

It was shown that up to 40 molecules of IgG could be conjugated to one single oil cationic droplet.

In conclusion, this galenic vector was shown to increase the number of antibody sites on AML cells and to improve the half-life of anti CD44 antibodies.

### Example 6: Treatment according to the invention

Formulation of the medicament (flask of 20mL, lyophilized):
- active principle: 100 mg of lyophilized P245
- excipients : saccharose, polysorbate, monosodic phosphate, disodic phosphate.

This formulation may be kept between +2 and +8°C, in its packaging for 18 months. Do not freeze.

Once prepared, the medicament may be preserved only 3 hours.

Treatment: 5mg/Kg are injected by slow infusion (for example, during two hours).

## Claims

1. Chimeric anti-CD44 antibody fragments, comprising F(ab')₂, Fab, Fab', scFv, Fv or CDRs fragments of a chimeric anti-CD44 antibody comprising amino acid sequences coded by nucleotidic sequences SEQ ID N°1, SEQ ID N°3 and SEQ ID N°5 or SEQ ID N°1, SEQ ID N°3 and SEQ ID N°7.

2. Chimeric anti-CD44 antibody fragments, comprising F(ab')₂, Fab, Fab', scFv, Fv or CDRs fragments of a chimeric anti-CD44 antibody comprising sequences SEQ ID N°2, SEQ ID N°4 and SEQ ID N°6 or SEQ ID N°2, SEQ ID N°4 and SEQ ID N°8 .

3. Fragments according to claim 1 or 2, wherein the fragments are coupled with a galenic vector.

4. Fragments according to anyone of claim 1 to 3, for use in the preparation of a medicament for eradicating pathological stem cells in cancer therapy.

5. Fragments according to claim 4, for use in the preparation of a medicament for specifically eradicating pathological stem cells and preserving normal stem cells ex vivo in cancer therapy.

6. Fragments according to claim 4 or 5, wherein said pathological stem cells are leukaemic stem cells.

7. Fragments according to anyone of claim 1 to 3, for use in the preparation of a medicament for differentiating leukaemic cells and for eradicating leukaemic stem cells.

8. Fragments according to anyone of claim 4 or 5, wherein said pathological stem cells are breast or colon cancer stem cells.

9. Fragments according to anyone of claims 4 to 8, which are administered by slow infusion at doses from 10mg to 1000mg by cure.

10. Nucleotide sequence selected from the SEQ ID N°1 and SEQ ID N°3.

11. Amino acid sequence selected from the group consisting of SEQ ID N°2 and SEQ ID N°4.

12. Transfer construct comprising at least a nucleotide sequence according to claim 10.


SEQUENCE LISTING

<110>  INSTITUT NATIONAL DE LA SANTE ET DE LA RECHERCHE MEDICALE
       UNIVERSITY HEALTH NETWORK

<120>  CHIMERIC ANTI CD44 ANTIBODIES AND THEIR USE FOR TREATING ACUTE
       MYELOID LEUKEMIA

<130>  CP/BB 61172-3569

<150>  EP 03 292 873.1
<151>  2003-11-19

<150>  EP 04 803 792.3
<151>  2004-11-19

<150>  PCT/EP04/014157
<151>  2004-11-19

<160>  8

<170>  PatentIn version 3.5

<210>  1
<211>  381
<212>  DNA
<213>  Artificial sequence

<220>
<223>  VL nucleotide sequence

<400>  1
gatgttgtga tgactcagac cccactcact ttgtcggtta ccattggaca accagcctcc      60

atctcttgca aatcaagtca gagcctctta catagtaatg gaaagacata tttgaattgg     120

ttgttacaga ggccaggcca gtctccaaag ctcctaatct atctggtgtc taaactggaa     180

tctggagtcc ctgacaggtt cagtggcagt ggatcaggga cagaattcac actgaaaatc     240

agcagagtgg aggctgagga ttcgggagtt tattactgct tgcaagctac acattttcct     300

ctcacgttcg gtgctgggac caaactggag ctgaaacggg ctgatgctgc accaactgta     360

tccatcttcc caccagtcga c                                               381


<210>  2
<211>  127
<212>  PRT
<213>  Artificial Sequence

<220>
<223>  VL amino acid sequence

<400>  2

Asp Val Val Met Thr Gln Thr Pro Leu Thr Leu Ser Val Thr Ile Gly
1               5                   10                  15


Gln Pro Ala Ser Ile Ser Cys Lys Ser Ser Gln Ser Leu Leu His Ser
            20                  25                  30


Asn Gly Lys Thr Tyr Leu Asn Trp Leu Leu Gln Arg Pro Gly Gln Ser
        35                  40                  45

Pro Lys Leu Leu Ile Tyr Leu Val Ser Lys Leu Glu Ser Gly Val Pro
    50                  55                  60

Asp Arg Phe Ser Gly Ser Gly Ser Gly Thr Glu Phe Thr Leu Lys Ile
65                  70                  75                  80

Ser Arg Val Glu Ala Glu Asp Ser Gly Val Tyr Tyr Cys Leu Gln Ala
                85                  90                  95

Thr His Phe Pro Leu Thr Phe Gly Ala Gly Thr Lys Leu Glu Leu Lys
            100                 105                 110

Arg Ala Asp Ala Ala Pro Thr Val Ser Ile Phe Pro Pro Val Asp
        115                 120                 125

<210>  3
<211>  448
<212>  DNA
<213>  Artificial Sequence

<220>
<223>  VH nucleotide sequence

<400>  3
caggttcagc tgcagcagtc tggggatgag ctggtgaggc ctgggtcctc agtgaagatt      60

tcctgcaagg cttctggcta tgcattcagt aggtactgga tgaactgggt gaagcagagg     120

cctggacagg gtcttgagtg gattggacag atttatcctg gagatggtga tactaactac     180

aatggaaagt tcaagggtaa agccacactg actgcagaca aatcctccag cacagcctac     240

atgcagctca acagcctaac atctgaggac tctgcggtct atttctgtgc aagacggcga     300

tggtccgatt actttggtat ggactactgg ggtcaaggaa cctcagtcac cgtctcctca     360

gccaaaacga cacccccatc tgtctatcca ctggcccctg gatctactgc ccaaactaac     420

tccatggtga ccctgggatg cctgggtc                                        448

<210>  4
<211>  149
<212>  PRT
<213>  Artificial Sequence

<220>
<223>  VH amino acid sequence

<400>  4

Gln Val Gln Leu Gln Gln Ser Gly Asp Glu Leu Val Arg Pro Gly Ser
1               5                   10                  15

Ser Val Lys Ile Ser Cys Lys Ala Ser Gly Tyr Ala Phe Ser Arg Tyr
            20                  25                  30

Trp Met Asn Trp Val Lys Gln Arg Pro Gly Gln Gly Leu Glu Trp Ile
        35                  40                  45

Gly Gln Ile Tyr Pro Gly Asp Gly Asp Thr Asn Tyr Asn Gly Lys Phe

50                  55                  60

Lys Gly Lys Ala Thr Leu Thr Ala Asp Lys Ser Ser Ser Thr Ala Tyr
65                  70                  75                  80

Met Gln Leu Asn Ser Leu Thr Ser Glu Asp Ser Ala Val Tyr Phe Cys
                85                  90                  95

Ala Arg Arg Arg Trp Ser Asp Tyr Phe Gly Met Asp Tyr Trp Gly Gln
            100                 105                 110

Gly Thr Ser Val Thr Val Ser Ser Ala Lys Thr Thr Pro Pro Ser Val
        115                 120                 125

Tyr Pro Leu Ala Pro Gly Ser Thr Ala Gln Thr Asn Ser Met Val Thr
    130                 135                 140

Leu Gly Cys Leu Gly
145

<210>  5
<211>  990
<212>  DNA
<213>  Artificial Sequence

<220>
<223>  Nucleotide gamma 1 constant region sequence

<400>  5
gctagcacca agggcccatc ggtcttcccc ctggcaccct cctccaagag cacctctggg      60

ggcacagcgg ccctgggctg cctggtcaag gactacttcc ccgaaccggt gacggtgtcg     120

tggaactcag gcgccctgac cagcggcgtg cacaccttcc cggctgtcct acagtcctca     180

ggactctact ccctcagcag cgtggtgacc gtgccctcca gcagcttggg cacccagacc     240

tacatctgca acgtgaatca caagcccagc aacaccaagg tggacaagaa agttgagccc     300

aaatcttgtg acaaaactca cacatgccca ccgtgcccag cacctgaact cctgggggga     360

ccgtcagtct tcctcttccc cccaaaaccc aaggacaccc tcatgatctc ccggacccct     420

gaggtcacat gcgtggtggt ggacgtgagc cacgaagacc ctgaggtcaa gttcaactgg     480

tacgtggacg gcgtggaggt gcataatgcc aagacaaagc cgcgggagga gcagtacaac     540

agcacgtacc gtgtggtcag cgtcctcacc gtcctgcacc aggactggct gaatggcaag     600

gagtacaagt gcaaggtctc caacaaagcc ctcccagccc ccatcgagaa aaccatctcc     660

aaagccaaag ggcagccccg agaaccacag gtgtacaccc tgcccccatc ccgggatgag     720

ctgaccaaga accaggtcag cctgacctgc ctggtcaaag gcttctatcc cagcgacatc     780

gccgtggagt gggagagcaa tgggcagccg gagaacaact acaagaccac gcctcccgtg     840

ctggactccg acggctcctt cttcctctac agcaagctca ccgtggacaa gagcaggtgg     900

cagcagggga acgtcttctc atgctccgtg atgcatgagg ctctgcacaa ccactacacg     960

cagaagagcc tctccctgtc tccgggtaaa                                      990

<210>  6
<211>  330
<212>  PRT
<213>  Artificial Sequence

<220>
<223>  Amino acid gamma 1 constant region sequence

<400>  6

<210>  7
<211>  981
<212>  DNA
<213>  Artificial Sequence

<220>
<223>  Nulceotide gamma 4 constant region sequence

<400>  7
gcttccacca agggcccatc cgtcttcccc ctggcgccct gctccaggag cacctccgag      60

agcacagcgg ccctgggctg cctggtcaag gactacttcc ccgaaccggt gacggtgtcg     120

tggaactcag gcgccctgac cagcggcgtg cacaccttcc cggctgtcct acagtcctca     180

ggactctact ccctcagcag cgtggtgacc gtgccctcca gcagcttggg cacgaagacc     240

tacacctgca atgtagatca caagcccagc aacaccaagg tggacaagag agttgagtcc     300

aaatatggtc ccccatgccc atcatgccca gcacctgagt tcctgggggg accatcagtc     360

ttcctgttcc ccccaaaacc caaggacact ctcatgatct cccggacccc tgaggtcacg     420

tgcgtggtgg tggacgtgag ccaggaagac cccgaggtcc agttcaactg gtacgtggat     480

ggcgtggagg tgcataatgc caagacaaag ccgcgggagg agcagttcaa cagcacgtac     540

cgtgtggtca gcgtcctcac cgtcctgcac caggactggc tgaacggcaa ggagtacaag     600

tgcaaggtct ccaacaaagg cctcccgtcc tccatcgaga aaaccatctc caaagccaaa     660

gggcagcccc gagagccaca ggtgtacacc ctgcccccat cccaggagga gatgaccaag     720

aaccaggtca gcctgacctg cctggtcaaa ggcttctacc ccagcgacat cgccgtggag     780

tgggagagca atgggcagcc ggagaacaac tacaagacca cgcctcccgt gctggactcc     840

gacggctcct tcttcctcta cagcaggcta accgtggaca agagcaggtg gcaggagggg     900

aatgtcttct catgctccgt gatgcatgag gctctgcaca accactacac acagaagagc     960

ctctccctgt ctctgggtaa a                                               981

<210>  8
<211>  327
<212>  PRT
<213>  Artificial Sequence

<220>
<223>  Amino acid gamma 4 constant region sequence

<400>  8

Ala Ser Thr Lys Gly Pro Ser Val Phe Pro Leu Ala Pro Cys Ser Arg
1               5                   10                  15

Ser Thr Ser Glu Ser Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Lys Thr
65                  70                  75                  80

Tyr Thr Cys Asn Val Asp His Lys Pro Ser Asn Thr Lys Val Asp Lys
                85                  90                  95

Arg Val Glu Ser Lys Tyr Gly Pro Pro Cys Pro Ser Cys Pro Ala Pro
            100                 105                 110

Glu Phe Leu Gly Gly Pro Ser Val Phe Leu Phe Pro Pro Lys Pro Lys
        115                 120                 125

Asp Thr Leu Met Ile Ser Arg Thr Pro Glu Val Thr Cys Val Val Val
    130                 135                 140

Asp Val Ser Gln Glu Asp Pro Glu Val Gln Phe Asn Trp Tyr Val Asp
145                 150                 155                 160

Gly Val Glu Val His Asn Ala Lys Thr Lys Pro Arg Glu Glu Gln Phe
                165                 170                 175

Asn Ser Thr Tyr Arg Val Val Ser Val Leu Thr Val Leu His Gln Asp
            180                 185                 190

Trp Leu Asn Gly Lys Glu Tyr Lys Cys Lys Val Ser Asn Lys Gly Leu
        195                 200                 205

Pro Ser Ser Ile Glu Lys Thr Ile Ser Lys Ala Lys Gly Gln Pro Arg
    210                 215                 220

Glu Pro Gln Val Tyr Thr Leu Pro Pro Ser Gln Glu Glu Met Thr Lys

225                 230                 235                 240

Asn Gln Val Ser Leu Thr Cys Leu Val Lys Gly Phe Tyr Pro Ser Asp
                245                 250                 255

Ile Ala Val Glu Trp Glu Ser Asn Gly Gln Pro Glu Asn Asn Tyr Lys
            260                 265                 270

Thr Thr Pro Pro Val Leu Asp Ser Asp Gly Ser Phe Phe Leu Tyr Ser
        275                 280                 285

Arg Leu Thr Val Asp Lys Ser Arg Trp Gln Glu Gly Asn Val Phe Ser
    290                 295                 300

Cys Ser Val Met His Glu Ala Leu His Asn His Tyr Thr Gln Lys Ser
305                 310                 315                 320

Leu Ser Leu Ser Leu Gly Lys
                325